(19) Europäisches Patentamt
European Patent Office
Office européen des brevets

(11) EP 2 027 849 A1

(12) EUROPEAN PATENT APPLICATION
published in accordance with Art. 153(4) EPC

(43) Date of publication:
**25.02.2009 Bulletin 2009/09**

(21) Application number: **07743888.5**

(22) Date of filing: **22.05.2007**

(51) Int Cl.:
***A61K 8/86*** *(2006.01)* ***A61K 47/34*** *(2006.01)*
***A61Q 1/02*** *(2006.01)* ***A61Q 17/04*** *(2006.01)*
***A61Q 19/00*** *(2006.01)*

(86) International application number:
**PCT/JP2007/060454**

(87) International publication number:
**WO 2007/136067 (29.11.2007 Gazette 2007/48)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **23.05.2006 JP 2006142384**
**29.09.2006 JP 2006269122**

(71) Applicants:
- **Shiseido Company, Limited**
  **Chuo-ku**
  **Tokyo 104-8010 (JP)**
- **NOF Corporation**
  **20-3 Ebisu 4-chome, Shibuya-ku**
  **Tokyo 150-6019 (JP)**

(72) Inventors:
- **MIYAHARA, Reiji**
  **Yokohama-shi, Kanagawa 224-8558 (JP)**
- **HIWATARI, Kouzou**
  **Kawasaki-shi, Kanagawa 216-0003 (JP)**
- **WATANABE, Kei**
  **Yokohama-shi, Kanagawa 224-8558 (JP)**
- **OKA, Takashi**
  **Yokohama-shi, Kanagawa 224-8558 (JP)**
- **MARUYAMA, Kei-ichi**
  **Kawasaki-shi Kanagawa 210-0865 (JP)**
- **YOKOZAWA, Keiichi**
  **Kawasaki-shi, Kanagawa 210-0865 (JP)**

(74) Representative: **Lippert, Stachow & Partner**
**Patentanwälte**
**Frankenforster Strasse 135-137**
**51427 Bergisch Gladbach (DE)**

# (54) EXTERNAL PREPARATION FOR SKIN

(57) The present invention is to provide a skin external preparation with good moisture retention, a rough skin improving effect, and an improved feeling in use.

A skin external preparation comprising a polyoxyalkylene glycol/polyethylene glycol copolymer alkyl ether derivative represented by the below-described general formula (I):

(formula I) $Y\text{-}\{O(EO)_a/(AO)_b\text{-}R\}_k$ (I)

(In the formula, Y is the residue given by removing hydroxyl groups from a polyhydric alcohol having 3 to 6 hydroxyl groups, and k is the number of hydroxyl groups of the polyhydric alcohol. EO is an oxyethylene group; AO is an oxyalkylene group having 3 to 4 carbon atoms, and $1 \leqq a \leqq 70$, $1 \leqq b \leqq 70$. The percentage of the oxyethylene groups with respect to the sum of the oxyalkylene groups having 3 to 4 carbon atoms and the oxyethylene groups is 20 to 100 mass %. The oxyalkylene group having 3 to 4 carbon atoms and the oxyethylene group are added randomly. Rs are either identical to or different from each other, and they are hydrocarbon groups having 1 to 4 carbon atoms.)

EP 2 027 849 A1

## Description

### RELATED APPLICATIONS

This application claims the priority of Japanese Patent Application No. 2006-142384 filed on May 23, 2006, and the Japanese Patent Application No. 2006-269122 dated on September 29, 2006,which is incorporated herein by reference.

### TECHNICAL FIELD

The present invention relates to a skin external preparation, and in particular, relates to good moisture retention, a rough skin improving effect, and an improved feeling in use of a skin external preparation containing polyoxyalkylene glycol/polyethylene glycol copolymer alkyl ether derivative as an active component.

### BACKGROUND ART

In order to maintain healthy skin, the retention of moisture is absolutely necessary, and numerous skin external preparations for the retention of moisture have been developed. As for the feeling in use, skin external preparations with a smooth feeling and no sticky feeling are sought-after.
Moisturizers have been actively researched, and the examples of moisturizers that are used in various applications such as lotion and milky lotion include glycerin. Glycerin has a rough skin improving effect in addition to a moisturizing effect. However, the blending quantity of glycerin needs to be increased in order to enhance a moisturizing effect and a rough skin improving effect. As a result, the following problems to be solved are generated. The system becomes unstable and the usability becomes poor. When applied on the skin, it is repelled by sebum and the compatibility with the skin becomes poor.
As moisturizers other than glycerin, polyols such as 1,3-butylene glycol, alkylene glycol, polyethylene glycol, sorbitol, and xylitol are known.
These polyols provide a low sticky feeling during use compared with glycerin; however, the following problems need to be solved. The moisturizing effect and rough skin improving effect are low. When applied on the skin, they are repelled by sebum, in the same way as glycerin, and the compatibility with the skin becomes poor.
On the other hand, (poly)ethylene glycol dialkyl ethers with a good feeling in use and with a good skin care effect are reported (for example, refer to patent literature 1). This (poly)ethylene glycol dialkyl ether is an oily base, and the oil can achieve a barrier function and the suppression of percutaneous water loss by the formation of a hydrophobic film. However, this (poly)ethylene glycol dialkyl ether itself cannot function as a moisturizer because of its low water solubility.
In order to solve these shortcomings, the present inventors have developed alkylene oxide derivatives (for example, refer to patent literature 2 and patent literature 3); however, further stickiness improvement has been desired.

Patent literature 1: Japanese Patent No.3658012
Patent literature 2: Japanese Patent No.3660650
Patent literature 3: Japanese Patent No.3667707

### DISCLOSURE OF THE INVENTION

### PROBLEM TO BE SOLVED BY THE INVENTION

The present invention was made in view of the above-described problems of the conventional technology, and the object of the invention is to provide a skin external preparation with good moisture retention, a rough skin improving effect, and an improved feeling in use.

### MEANS TO SOLVE THE PROBLEM

The present inventors have diligently studied; as a result, the present inventors have found that a skin external preparation excellent in the texture during use, especially in smoothness, without a sticky feeling, and with a moisturizing effect and a rough skin improving effect could be obtained by blending a specific polyoxyalkylene glycol/polyethylene glycol copolymer alkyl ether derivative in the skin external preparation. The present inventors have also found that, by using a polyoxyalkylene glycol/polyethylene glycol copolymer alkyl ether derivative in combination with moisturizers such as glycerin, the percutaneous absorption of the moisturizer could be promoted, the moisturizing effect and the

rough skin improving effect could be synergistically and markedly improved, and the stickiness due to the moisturizer could be improved. It was also found that polyoxyalkylene glycol/polyethylene glycol copolymer alkyl ether derivatives could achieve a percutaneous absorption promoting effect for whitening agents such as arbutin, thus leading to completion of the present invention.

Thus, the skin external preparation of the present invention is characterized in that a polyoxyalkylene glycol/polyethylene glycol copolymer alkyl ether derivative represented by the below-described general formula (I) is contained.

(formula I) $Y\text{-}\{O(EO)_a/(AO)_b\text{-}R)_k$ (I)

(In the formula, Y is the residue given by removing hydroxyl groups from a polyhydric alcohol having 3 to 6 hydroxyl groups, and k is the number of hydroxyl groups of the polyhydric alcohol. EO is an oxyethylene group; AO is an oxyalkylene group having 3 to 4 carbon atoms, and $1 \leqq a \leqq 70$, $1 \leqq b \leqq 70$. The percentage of the oxyethylene groups with respect to the sum of the oxyalkylene groups having 3 to 4 carbon atoms and the oxyethylene groups is 20 to 100 mass %. The oxyalkylene group having 3 to 4 carbon atoms and the oxyethylene group are added randomly. Rs are either identical to or different from each other, and they are hydrocarbon groups having 1 to 4 carbon atoms.)

In the above-described general formula (I) of the present invention, it is desirable that Y is the residue of pentaerythritol and that the percentage of the oxyethylene groups with respect to the sum of the oxyalkylene groups having 3 to 4 carbon atoms and the oxyethylene groups is 50 to 80 mass %.
It is also desirable that 0.01 to 70 mass % of a polyoxyalkylene glycol/polyethylene glycol copolymer alkyl ether derivative represented by the above-described general formula (I) is contained in the skin external preparation of the present invention.

In the moisturizer of the present invention, a polyoxyalkylene glycol/polyethylene glycol copolymer alkyl ether derivative represented by the above-described general formula (I) is an active component.
In the rough skin improving agent of the present invention, a polyoxyalkylene glycol/polyethylene glycol copolymer alkyl ether derivative represented by the above-described general formula (I) is an active component.

In the stickiness improving agent of the present invention, a polyoxyalkylene glycol/polyethylene glycol copolymer alkyl ether derivative represented by the above-described general formula (I) is an active component.
In the percutaneous absorption promoter of the present invention, a polyoxyalkylene glycol/polyethylene glycol copolymer alkyl ether derivative represented by the above-described general formula (I) is an active component.

The skin external preparation of the present invention is characterized in that it comprises a polyoxyalkylene glycol/polyethylene glycol copolymer alkyl ether derivative represented by the general formula (I) and a hydrophilic agent and that the polyoxyalkylene glycol/polyethylene glycol copolymer alkyl ether derivative is a percutaneous absorption promoter of the hydrophilic agent.
It is desirable that the hydrophilic agent is a moisturizer and that the moisturizer is glycerin or xylitol.
It is also desirable that the hydrophilic agent is at least one selected from the group consisting of hydroquinone derivatives and ascorbic acid derivatives.

In the percutaneous absorption control agent of the present invention, a polyoxyalkylene glycol/polyethylene glycol copolymer alkyl ether derivative represented by the above-described general formula (I) is an active component.

## EFFECT OF THE INVENTION

By blending a specific polyoxyalkylene glycol/polyethylene glycol copolymer alkyl ether derivative, the following can be achieved by the skin external preparation of the present invention. Firstly, the skin external preparation becomes excellent in texture during use, especially in smoothness, and the sticky feeling disappears. Secondly, the skin external preparation can achieve a moisturizing effect and rough skin improving effect. With the combined use of a moisturizer, the polyoxyalkylene glycol/polyethylene glycol copolymer alkyl ether derivative promotes the stratum corneum penetration of the moisturizer, and the moisturizing effect and rough skin improving effect can be significantly improved. In addition, the polyoxyalkylene glycol/polyethylene glycol copolymer alkyl ether derivative promotes the percutaneous absorption of whitening agents.

## BRIEF DESCRIPTION OF THE DRAWINGS

Fig.1 shows the percutaneous absorption promoting effect, for glycerin, of a polyoxyalkylene glycol/polyethylene glycol copolymer alkyl ether derivative of the present invention.
Fig.2 shows the percutaneous absorption promoting effect, for xylitol, of a polyoxyalkylene glycol/polyethylene glycol copolymer alkyl ether derivative of the present invention.

Fig.3 shows phase diagrams in which the lowering effect of the surfactant association number is compared between a polyoxyalkylene glycol/polyethylene glycol copolymer alkyl ether derivative of the present invention and a linear alkylene oxide derivative.

## BEST MODE FOR CARRYING OUT THE INVENTION

There is a performance difference between the polyoxyalkylene glycol/polyethylene glycol copolymer alkyl ether derivatives, which are characterized in the present invention and represented by the above-described general formula (I), and the alkylene oxide derivatives, which were previously reported by the present inventors and represented by the below-described general formula (II). That is, the sterically-bulky polyoxyalkylene glycol/polyethylene glycol copolymer alkyl ether derivatives have a strong function to lower the association number than the linear alkylene oxide derivatives, and the prevention effect of the viscosity increase becomes high. Accordingly, if a polyoxyalkylene glycol/polyethylene glycol copolymer alkyl ether derivative of the present invention is blended in the skin external preparation, the frictional feeling and sticky feeling can be effectively improved.

(formula II) $R^1O\text{-}[(AO)_m (EO)_n]\text{-}R^2$ (II)

(In the formula, AO is an oxyalkylene group having 3 to 4 carbon atoms, EO is an oxyethylene group, and m and n are the average addition mole numbers of the oxyalkylene group and the oxyethylene group, respectively, and $1 \leqq m \leqq 70$, $1 \leqq n \leqq 70$. The percentage of the oxyethylene groups with respect to the sum of the oxyalkylene groups having 3 to 4 carbon atoms and the oxyethylene groups is 20 to 80 mass %. The oxyalkylene group having 3 to 4 carbon atoms and the oxyethylene group may be added either in a block type or in a random type. $R^1$ and $R^2$ are either identical to or different from each other; they are either hydrocarbon groups having 1 to 4 carbon atoms or hydrogen atoms, and the ratio of the number of hydrogen atoms with respect to the number of hydrocarbon groups, $R^1$ and $R^2$, is 0.15 or less.)

In the polyoxyalkylene glycol/polyethylene glycol copolymer alkyl ether derivative represented by the characteristic general formula (I) of the present invention, AO is an oxyalkylene group having 3 to 4 carbon atoms, and the specific examples include an oxypropylene group, oxybutylene group, oxyisobutylene group, oxytrimethylene group, and oxytetramethylene group; preferably the oxyalkylene group is an oxypropylene group or oxybutylene group.
The average addition mole number of the oxyethylene groups is represented by a, and $1 \leqq a \leqq 70$, preferably $2 \leqq a \leqq 20$. The average addition mole number of the oxyalkylene groups having 3 to 4 carbon atoms is represented by b, and $1 \leqq b \leqq 70$, preferably $2 \leqq b \leqq 0$. Here, the values a and b are either identical to or different from each other. If the number of the oxyalkylene groups having 3 to 4 carbon atoms or the number of the oxyethylene groups is zero, the moist feeling decreases. If the number of the oxyalkylene groups having 3 to 4 carbon atoms or the number of the oxyethylene groups exceeds 70, a sticky feeling is generated and a satisfactory smooth feeling cannot be achieved.

In the present invention, the compounds obtained by randomly adding ethylene oxide and alkylene oxide having 3 to 4 carbon atoms are used.
In general formula (I), Y is the residue given by removing hydroxyl groups from a polyhydric alcohol having 3 to 6 hydroxyl groups, and k is the number of hydroxyl groups of the polyhydric alcohol and k is 3 to 6. Examples of the compounds having 3 to 6 hydroxyl groups include glycerin, trimethylolpropane, and <u>hexylene glycol</u> (k = 3); erythritol and pentaerythritol (k = 4); xylitol (k = 5); and sorbitol and inositol (k = 6). The basic skeleton of the polyoxyalkylene glycol/polyethylene glycol copolymer alkyl ether derivative, which is blended in the skin external preparation of the present invention, is the residue given by removing hydroxyl groups from a mixture of one or more polyhydric alcohols having 3 to 6 hydroxyl groups.
In the present invention, it is preferable that Y is the residue given by removing hydroxyl groups from a polyhydric alcohol having 3 to 4 hydroxyl groups, namely, it is desirable to satisfy $3 \leqq k \leqq 4$. If k is 2 or lower, a smooth feeling tends to be poor when blended in the skin external preparation. If k is 7 or higher, a sticky feeling tends to be generated. More preferably, Y is pentaerythritol and k = 4.

R is a hydrocarbon group having 1 to 4 carbon atoms or a hydrogen atom. Examples of hydrocarbon groups include a methyl group, ethyl group, n-propyl group, isopropyl group, n-butyl group, sec-butyl group, and tert-butyl group. Preferably it is a methyl group or ethyl group. In the case of hydrocarbon groups having 5 or more carbon atoms, the hydrophilicity decreases and the moist feeling decreases.
Each R may be of only one kind, or a mixture of different hydrocarbon groups having 1 to 4 carbon atoms may be present.

In the synthesis process of the polyoxyalkylene glycol/polyethylene glycol copolymer alkyl ether derivative of the present invention, a side chain in which R is a hydrogen atom may also be prepared. Among the hydrocarbon groups R in the present invention, however, the ratio of the number of hydrogen atoms (Y) with respect to the number of hydrocarbon groups (X),Y/X, is 0.15 or lower, and preferably 0.06 or lower. If the ratio of Y/X exceeds 0.15, a sticky feeling is generated.
The percentage of the total EO in the above-described formula (I) with respect to the sum of AO and EO in the above-described formula (I) is preferably 20 to 100 mass %, and more preferably 20 to 90 mass %. Most preferably, the percentage is 50 to 80 mass %. If the percentage is lower than 20 mass %, the moist texture during use tends to be poor.

The polyoxyalkylene glycol/polyethylene glycol copolymer alkyl ether derivative of the present invention can be prepared by a publicly known method. For example, it can be obtained by the addition polymerization, to pentaerythritol, of ethylene oxide and alkylene oxide having 3 to 4 carbon atoms and by the subsequent etherification, in the presence of an alkaline catalyst, with alkyl halide.

The blending quantity of the polyoxyalkylene glycol/polyethylene glycol copolymer alkyl ether derivative in the skin external preparation of the present invention is not limited in particular. The blending quantity is normally about 0.01 to 70 mass %, and preferably about 0.5 to 40 mass %. If the blending quantity is less than 0.01 mass %, the manifestation of the blending effect may not be satisfactory. If the blending quantity exceeds 70 mass %, stickiness may be felt after use.

The polyoxyalkylene glycol/polyethylene glycol copolymer alkyl ether derivative of the present invention exhibits a moisturizing effect and rough skin improving effect when blended in the skin external preparation. When other moisturizers such as glycerin are blended, a stickiness suppression effect is achieved. Examples of co-existable moisturizers include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salts, dl-pyrrolidone carboxylates, short-chain soluble collagen, diglycerin (EO)PO adduct, chestnut rose extract, yarrow extract, and melilot extract.

The polyoxyalkylene glycol/polyethylene glycol copolymer alkyl ether derivative (I) of the present invention also has a promoting effect of stratum corneum penetration of other moisturizers. Accordingly, if a polyoxyalkylene glycol/polyethylene glycol copolymer alkyl ether derivative (I) and other moisturizers are used in combination in the skin external preparation, an extremely high moisturizing effect and rough skin improving effect can be achieved because of the synergistic effect. As such moisturizers, there are moisturizers described above, and glycerin and xylitol are especially desirable.

The blending quantity of a moisturizer is not limited in particular, preferably 0.001 to 20.00 mass % of the total skin external preparation, and more preferably 0.1 to 10.0 mass %.

The polyoxyalkylene glycol/polyethylene glycol copolymer alkyl ether derivative (I) of the present invention also has a promoting effect of stratum corneum penetration of whitening agents, such as arbutin, which are other hydrophilic agents other than moisturizers.

The skin external preparation of the present inventions can be prepared by blending the above-described essential components to an existing skin external preparation base. In the skin external preparations of the present invention, other components normally used in the skin external preparations, such as cosmetics and pharmaceuticals, can be blended as necessary in addition to the above-described essential components. Examples of other components include powder components, liquid fat, solid fat, wax, hydrocarbons, higher fatty acids, higher alcohols, esters, silicones, anionic surfactants, cationic surfactants, amphoteric surfactants, nonionic surfactants, water-soluble polymers, thickeners, film-forming agents, UV absorbers, metal ion sequestering agents, lower alcohols, polyhydric alcohols, saccharides, amino acids, organic amines, polymer emulsions, pH adjusters, skin nutrients, vitamins, antioxidants, antioxidant promoters, perfumes, and water, and normal preparation methods can be used in accordance with desired product forms. Specific components that can be blended are listed in the following. The skin external preparations of the present invention can be prepared by blending the above-described essential components and any one or more of the below-described components.

As powder components, for example, inorganic powder (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate, calcium phosphate, fluorine apatite, hydroxyapatite, ceramic powder, metallic soap (for example, zinc myristate, calcium palimitate, and aluminum stearate), and boron nitride, etc); organic powder (for example, polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, etc); inorganic white family pigment (for example, zinc oxide, etc); inorganic red family pigment (for example, iron oxide (colcothar), and iron titanate, etc); inorganic brown family pigment (for example, γ-iron oxide, etc); inorganic yellow family pigment (for example, yellow iron oxide, and loess, etc); inorganic black family pigment (for example, black iron oxide, and lower titanium oxide, etc); inorganic purple family pigment (for example, mango violet, cobalt violet, etc); inorganic green family pigment (for example, chrome oxide, chrome hydroxide, cobalt titanate, etc); inorganic blue family pigment (for example, ultramarine, iron blue, etc); pearl pigment (for example, titanium oxide coated mica, titanium oxide coated bismuth oxychloride, titanium oxide coated talc, colored titanium oxide coated mica, bismuth oxychloride, argentine, etc); metal powder pigment (aluminum powder, copper powder, etc); organic pigment such as zirconium, barium, or aluminum lake (for example, organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401,or Red No.3, Red No.104, Red No.106, Red No.227, Red No.230, Red No.401,

Red No.505, Orange No.205, Yellow No.4, Yellow No.5, Yellow No.202, Yellow No.203, Green No.3, and Blue No.1, etc); natural pigment (for example, chlorophyll, β-carotene, etc), etc.

As liquid fat, for example, avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, chinese wood oil, jojoba oil, germ oil, triglycerol, can be listed.

As solid fat, for example, cacao butter, coconut oil, horse fat, hydrogenated coconut oil, palm oil, beef fat, mutton suet, hydrogenated beef fat, palm kernel oil, lard, beef bones fat, Japan wax kernel oil, hardened oil, hoof oil, Japan wax, hydrogenated caster oil, can be listed.

As waxes include ,for example, beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, and POE hydrogenated lanolin alcohol ether, can be listed.

As hydrocarbon oils include liquid paraffin, ozocerite, squalene, pristane, paraffin, ceresin. squalane, vaseline, microcrystalline wax, can be listed. As higher fatty asid include, for example, lauric acid, myristic acid, palmitic acid, stearic acid, behenic acid, oleic acid, undecylenic acid, tallic acid, isostearic acid, linoleic acid, linolenic acid, eicosapentaenoic acid(EPA), docosahexaenoic acid(DHA) and the like.

Higher alcohol include, for example, linear alcohol (for example, lauryl alcohol, cetyl alcohol, stearyl alcohol, behenyl alcohol, myristyl alcohol, oleyl alcohol, and cetostearyl alcohols; branched-chain alcohols (for example, monostearylglycerin ether (batyl alcohol),2-decyltetradecinol, lanolin alcohol, cholesterol, phytosterol, hexyldodecanol, isostearyl alcohol, and octyldodecanol) and the like.

As synthesis ester oils, isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoiso stearate, neopentyl glycol dicaprate, diisostearyl malate, glycerol di-2-heptyl undecanoate, trimethyrol propane tri-2-ethyl hexanoate, trimethyrol propane triisostearate, tetra-2-ethyl hexanoate pentaerythritol, glycerol tri-2-ethyl hexanoate, glycerol trioctanoate, glycerol triisopalmitate, trimethyrol propane triisostearate, cetyl 2-ethylhexanoate, 2-ethylhexyl palmitate, glycerol trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate can be listed.

Silicone oil include, for example, chain polysiloxane (for example, dimethylpolysiloxane, methylphenylpolysiloxane, diphenylpolysiloxane); cyclic polysiloxane (for example, octamethylcyclotetrasiloxane, decamethylcyclopentasiloxane, dodecamethylcyclohexasiloxane), silicone resins having a three dimensional network structure, silicone rubbers, various modified polysiloxanes (for example, amino-modified polysiloxane, polyether-modified polysiloxane, alkyl-modified polysiloxane, fluorine-modified polysiloxane) and the like.

Anionic surfactants include, for example, fatty acid soap (for example, sodi um laurate, sodium palmitate); higher alkyl sulfate ester salt (for example, sodium laur yl sulfate, potassium lauryl sulfate); alkyl ether sulfate ester salt (for example, POE la uryl sulfate triethanolamine, sodium POE lauryl sulfate); N-acyl sarcosinic acid ( for ex ample, sodium lauroyl sarcocinate ) ; higher fatty acid amide sulfonate (for example, sodium N-myristoyl-N-methyl taurine, sodium coconut oil fatty acid methyl tauride, so dium laurylmethyl tauride); phosphate ester salt (sodium POE oleylether phosphate, PO E stearylether phosphate); sulfosuccinate (for example, sodium di-2-ethylhexyl sulfosucc inate, sodium monolauroyl monoethanolamide polyethylene sulfosuccinate, sodium laury 1 polypropylene glycol sulfosuccinate); alkylbenzene sulfonate (for example, sodium lin ear dodecylbenzene sulfonate, triethanolamine linear dodeylbenzene sulfonate, linear do decylbenzene sulfonate); higher fatty acid ester sulfate ester salt (for example, sodium hydrogenated gryceryl cocoate sulfate), N- acyl glutamate (for example, monosodium N-lauroyl glutamate, disodium N-stearoyl glutamate, monosodium N-myristoyl-L-glutam ate); sulfonated oil (for example, Turkey red oil); POE alkyl ether carboxylic acid; P OE alkyl aryl ether carboxylate; α-olefine sulfonate; higher fatty acid ester sulfonate, s econdary alcohol sulfate ester salt, higher fatty acid alkylolamide sulfate ester salt, sod ium lauroyl monoethanolamide succinate; N-palmitoyl asparaginate ditriethanolamine; so dium casein and the like.

Cationic surfactants include, for example, alkyltrimethyl ammonium salt (for example, stearyltrimethyl ammonium chloride, lauryltrimethyl ammonium chloride); alk ylpyridinium salt (for example, cetylpyridinium chloride); distearyldimethyl ammonium chloride; dialkyldimethyl ammonium salt; poly (N,N'-dimethyl-3,5-methylenepiperidiniu m) chloride; alkyl quaternary ammonium salt; alkyldimethylbenzyl ammonium salt; alk ylisoquinolinium salt; dialkylmorphonium salt; POE alkylamine; alkylamine salt; polya mine fatty acid derivative; amyl alcohol fatty acid derivative; benzalkonium chloride; b enzethonium chloride and the like.

Ampholytic surfactants include, for example, imidazoline base ampholytic su rfactants (for example, sodium 2-undecyl-N,N,N-(hydroxyethylcarboxymethyl)-2-imidazol ine, 2-cocoyl-2-imidazolinium hydroxide-1-carboxyethyloxy)-2-sodium salt; betaine base surfactants (for example, 2-heptadecyl-N-carboxymethyl-N-hydroxyethylimidazolinium be taine, lauryldimethyl aminoacetate betaine, alkyl betaine, amidobetaine, sulfobetaine) an d the like.

Lipophilic nonionic surfactants include for example, sorbitan fatty acid ester s (for example, sorbitan monooleate, sorbitan monoisostearate, sorbitan monolaurate, so rbitan monopalmitate, sorbitan monostearate, sorbitan sesquioleate, sorbitan trioleate, dig lycerol sorbitan penta-2 ethylhexylate, diglycerol sorbitan tetra-2 ethylhexylate); glycery 1 polyglyceryl fatty acids (for example, glyceryl monocotton oil fatty acid, glyceryl mo noerucate, glyceryl sesquioleate, glyceryl monostearate, glyceryl α, α'-oleate pyroglutam ate, glyceryl monostearate malate); propylene glycol fatty acid esters (for example, pro pylene glycol monostearate); hydrogenated caster oil derivative; glyceryl alkyl ether an d the like.

Hydrophilic nonionic surfactants include, for example, POE sorbitan fatty acid esters (for example, POE sorbitan monooleate, POE sorbitan monostearate, POE sorbitan monooleate, POE sorbitan tetraoleate); POE sorbit fatty acid esters (for example, POE sorbit monolaurate, POE sorbit monooleate, POE sorbit pentaoleate, POE sorbit monostearate), POE glyceryl fatty acid esters (for example, POE monooleate such as POE glyceryl monostearate, POE glyceryl monoisostearate, POE glyceryl triisostearate); POE fatty acid esters (for example, POE distearate, POE monodioleate, ethyleneglycol distearate); POE alkyl ethers (for example, POE lauryl ether, POE oleyl ether, POE stearyl ether, POE behenyl ether, POE-2-octyldodecyl ether, POE cholestanol ether); puluronic types (for example, Puluronic), POE/POP alkyl ethers (for example, POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (for example, Tetronic); POE castor oil hydrogenated castor oil derivatives (for example, POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivatives (for example, POE sorbitol beeswax); alkanolamides (for example, coconut oil fatty acid diethanolamide, lauric acid monoethanolamide, fatty acid isopropanolamide); POE propyleneglycol fatty acid esters; POE alkyl amines; POE fatty acid amides; sucrose fatty acid esters; alkylethoxydimethylamine oxide; trioleyl phosphoric acid and the like.

As natural water-soluble polymer include, for example, plant-based polymer (for example, gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat), glicyrrhizic acid), microorganisms based polymer (for example, xanthan gum, dextran, succinoglycan, pullulan, etc), animal-based polymer (for example, collagen, casein, albumin, gelatine, etc) and the like.

As semisynthetic water-soluble polymer include, for example, starch-based polymer (for example, carboxymethyl starch, methylhydroxypropyl starch, etc), cellulosic polymer (methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, micrclrystalline cellulose, cellulose powder, etc), algin acid base polymer, (for example, alginate sodium, propylene glycol ester alginate, etc), and the like.

As synthetic water-soluble polymer include, for example, vinyl base polymer (for example, polyvinyl alcohol, polyvinyl methyl ether, polyvinylpyrrolidone, carboxyvinylpolymer, etc); polyoxyethylene base polymer (for example, polyethylene glycol 20,000, polyoxyethylenepolioxypropylene copolymer of 20,000 and 60,000); acrylic polymer (fro example, sodium polyacrylate, polyethylacrylate, polyacrylamide, etc); polyethyleneimine; cationpolymer, and the like.

As plasticizer include, for example, gum Arabic, carrageenan, gum karaya, gum tragacanth, guar gum, gum Arabic, locust bean gum, quince seed (cydonia oblonga), casein, dextrine, gelatine, sodium pectate, alginate sodium, methylcellulose, CMC, hydroxyethylcellulose, hydroxypropylcellulose, PVA, PVM, PVP, sodium polyacrylate, carboxyvinylpolymer, locust bean gum, guar gum, tamarind gum, dialkyldimethylammonium cellulose sulfate, xanthan gum, aluminium magnesium silicate, bentonite, hectorite, aluminium magnesium silicate (veegum), laponite, silicic anhydride, and the like.

Examples of ultraviolet light absorbers include benzoic acid family ultraviolet light absorbers (for example, p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid family ultraviolet light absorbers (for example, homomenthyl N-acetylanthranilate etc.); salicylic acid family ultraviolet light absorbers (for example, amyl salicylate, menthyl salicylate, homomenthyl salicylate, octyl salicylate, phenyl salicylate, benzyl salicylate, p-isopropanolphenyl salicylate, etc.); cinnamic acid family ultraviolet light absorbers (for example, octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc.); benzophenone family ultraviolet light absorbers (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.); 3-(4'-methylbenzylidene)-d,1-camphor and 3-benzylidene-d,1-camphor; 2-phenyl-5-methylbenzoxazol; 2,2'-hydroxy-5-methylphenylbenzotriazol, 2-(2'-hydroxy-5'-t-octylphenyl) benzotriazol, and 2-(2'-hydroxy-5'-methylphenylbenzotriazol; dibenzalazine; dianisoylmethane; 4-methoxy-4'-t-butyldibenzoylmethane; and 5-(3,3-dimethyl-2-norbornylidene)-3-pentane-2-one.

As chelate agents include, for example, 1-hydroxyethane-1, 1-diphosphonic acid, 1-hydroxyethane, 1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

As lower alcohol include, for example, ethanol, propanol, isopropanol, isobutyl alcohol, t-butyl alcohol, and the like.

As polyhydric alcohol include, for example, dihydric alcohol (for example, ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (for example, glycerin, trimethylolpropane, etc); tetrahydric alcohol (for example, such as pentaerythritol such as 1,2,6-hexanetriol); pentahydric alcohol (for example, xylitol, etc); hexahydric alcohol (for example, sorbitol, mannitol, etc); polyhydric alcohol polymer (for example, diethylene glycol, triethylene glycol, polypropylene glycol, tetraethylene glycol, diglycerin, polyethylene glycol, triglycerin, tetraglycerin, polyglycerin, etc); dihydric alcohol alkyl ethers (for example, ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (for example, diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (for example, ethylene glycol monomethyl ether acetate, ethylene glycol monobutyl ether acetate, ethylene glycol monophenyl ether acetate, ethylene glycol diadipate, ethylene glycol disaccinate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (for example, chimil alcohol, selachyl alcohol, batyl alcohol, etc); sugar alcohol (for example, sorbitol, maltitol, maltotriose, mannitol, sucrose, erythritol, glucose, fructose, starch sugar, maltose, xylitose, starch sugar hydrogenated alcohol, etc); <u>glysolid</u>, tetrahydrofurfuryl alcohol; POE-tetrahydrofurfuryl alcohol; POP-POE-butyl ether; tripolyoxypropylene glycerin ether; POP- glycerin ether; POP- glycerin ether phosphoric acid; POP-POE-pentaerythritol ether; polyglycerin, and the like.

As monosaccharides include, for example, triose (for example, D-glyceryl aldehyde, dihydroxyacetone, etc); tetrose (for example, D-erythrose, D- erythrulose, D-threose, erythritol, etc); pentaose (for example, L-arabinose, D-xylose, L-lyxose, D-arabinose, D-ribose, D-ribulose, D-xylulose, L-xylulose, etc); hexalose (for example, D-glucose, D-talose, D-psicose, D-galactose, D-fructose, L-galactose, L-mannose, D-tagatose, etc); heptose (for example, aldoheptose, heplose); octose (for example, octulose); deoxy sugar (for example, 2-deoxy-D-ribose, 6-deoxy-L- galactose, 6-deoxy-L-mannose); amino sugar (for example, D-glucosamine, D-galactosamine, sialic acid, amino uronic acid, muramic acid, etc); uronic acid (for example, D-grucuronic acid, D-mannuronic acid, L-guluronic acid, garacturonic acid, L-iduronic acid, etc) and the like.

As oligosaccharide include, for example, sucrose, guntianose, umbelliferose, lactose, planteose, <u>isolignose type</u>, $\alpha,\alpha$-trehalose, raffinose, <u>lignose type</u>, <u>umbellicine,</u> stachyose, verbascose type, and the like. As polysaccharide include, for example, cellulose, quince seed, chondroitinsulfate, starch, galactan, dermatan sulfate, glycogen, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

As amino acids include, for example, neutral amino acid (for example threonine, cysteine, etc); basic amino acid (for example, hydroxylysine, etc) and the like. As amino acid derivatives include, for example, sodium acyl sarcosine (sodium lauroyl sarcosine), acyl glutamate, sodium acyl $\beta$-alanine, glutathione, pyrrolidone carboxylate, and the like.

As the orgaminc amine include, for example, monoethanolamine, diethanolamine, morpholine, triisopropanolamine, 2-amino-2-methyl-1,3-propanediol, 2-amino-2-methyl-1-propanol, and the like. As polymer emulsion include, for example, acrylate resin emulsion, ethyl polyacrylate emulsion, liquid acryl resin, polyacrylalkylester emulsion, polyvinyl acrylate resin emulsion, natural rubber latex, and the like.

As pH modifiers include ,buffers such as lactic acid-sodium lactic acid, citric acid-sodium citric acid, succinic

acid- sodium succinic acid and the like. As vitamine group include, for example, vitamineA, B1, B2, B6, C, E, and their derivatives, pantothenic acid, and their derivatives, biotin and the like. As anti-oxidants include, tocopherols, dibutyl hydroxy toluene, butyl hydroxy anisole, and gallic acid esters, and the like.

As anti-oxidant aids include, for example, phosphoric acid, citric acid, ascorbic acid, maleic acid, malonic acid, succinic acid, fumaric acid, cephalin, hexamethaphosphate, phytic acid, and ethylene diamine tetra-acetic acid, and the like.

As other containable compositions include, for example, antiseptic agent (ethylparaben, butylparaben, etc); antiphlogistics (for example, glycyrrhizinic acid derivatives, salicylic acid derivatives, hinokitiol, zinc oxide, allantoin, etc), lightening agent (for example, placental extract, saxifrage extract, arbutin, etc); various extract (for example, cork tree bark, Japanese coptis, lithospermum, peony, swertia herb, birch, sage, loquat, carrot, aloe, mallow, iris, grape, mugwort, sponge gourd, lily, saffron, cnidium rhizome, ginger, hypericum, restharrow, garlic, red pepper, citrus unshiu, Japanese angelica, seaweed, etc); activator agent (for example, royal jelly, photosenstizer, cholesterol derivatives); blood circulation promotion agent (for example, nonyl acid vanillyl amide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc), antiseborrheric agent,( for example, sulfur, thianthl, etc); anti-inflammatory agent (for example, tranexamic acid, thiotaurine, hypotaurine, etc) and the like.

The skin external preparations of the present invention can be in any form such as a solution form, solubilized form, emulsion form, dispersed powder form, water-oil double-layer form, water-oil-powder triple-layer form, gel, mist, spray, mousse, roll-on, or stick. Formulations in which the skin external preparation is impregnated in or coated on a nonwoven sheet and the like are also possible. The products of the skin external preparations of the present invention can be in any form, and the examples include facial cosmetics such as lotion, milky lotion, cream, and packs; makeup cosmetics such as foundation, lipstick, and eye shadow; sunscreen cosmetics (sunscreen agent); body cosmetics; aromatic cosmetics; skin cleansers such as makeup remover and body shampoo; hair care cosmetics such as hair liquid, hair tonic, hair conditioner, shampoo, rinse, and hair growth promoter; and ointments.

## EXAMPLES

[Embodiment 1]

Hereinafter, examples of the present invention will be described.
Prior to the description of the examples of the present invention, the evaluation methods will be shown below.

"Testing by Conductance Measurement"

The skin conductance was measured before the application and after 30 minutes, 60 minutes, and 120 minutes of the application on the forearms of 10 panelists. From the rate of change, the moisturizing effect was evaluated. The rate of change of the skin conductance is determined by the following equation (III), and the influences on the water-absorbing property and the moisture-retention ability of the stratum corneum can be investigated. If the rate of change is small, it can be estimated that there is an increase in the water content in the stratum corneum and that the moisturizing effect is high.

Equation (III)

$$\text{Rate of conductance change} = (\text{conductance before application})/(\text{conductance after application})$$

The evaluation criteria for the "Testing by Conductance Measurement" are as follows.

◎: Average of the rate of conductance change of 10 panelists: 0 or higher and lower than 0.1
○: Average of the rate of conductance change of 10 panelists: 0.1 or higher and lower than 0.2
△: Average of the rate of conductance change of 10 panelists: 0.2 or higher and lower than 0.5
×: Average of the rate of conductance change of 10 panelists: 0.5 or higher

"Evaluation (1): Smoothness of the Skin"

The actual usage test by 10 professional panelists was conducted for the smoothness of the skin during use and after use. The evaluation criteria are as follows.

◎: 8 or more professional panelists recognized that the skin was smooth during use and after use.
○: 6 or more and less than 8 professional panelists recognized that the skin was smooth during use and after use.
△: 3 or more and less than 6 professional panelists recognized that the skin was smooth during use and after use.
×: less than 3 professional panelists recognized that the skin was smooth duri ng use and after use.

"Evaluation (2): Non-Stickiness on the Skin"

The actual usage test by 10 professional panelists was conducted for the non-stickiness on the skin during use and after use. The evaluation criteria are as follows.

◎: 8 or more professional panelists recognized that there was no stickiness on the skin during use and after use.
○: 6 or more and less than 8 professional panelists recognized that there was no stickiness on the skin during use and after use.
△: 3 or more and less than 6 professional panelists recognized that there was no stickiness on the skin during use and after use.
×: less than 3 professional panelists recognized that there was no stickiness o n the skin during use and after use.

"Evaluation (3): Moisturizing Effect Feeling"

The actual usage test by 10 professional panelists was conducted for the presence or absence of a moisturizing effect feeling after 120 minutes of use. The evaluation criteria are as follows.

◎: 8 or more professional panelists recognized that there was a moisturizing effect feeling.
○: 6 or more and less than 8 professional panelists recognized that there was a moisturizing effect feeling.
△: 3 or more and less than 6 professional panelists recognized that there was a moisturizing effect feeling.
×: less than 3 professional panelists recognized that there was a moisturizing effect feeling.

"Evaluation (4): Test for Rough Skin Improving Effect"

The test for a rough skin improving effect was conducted by 10 panelists having rough skin on the face (region: cheeks). The test method was as follows; different lotions were applied on the right and left cheeks for a week, and the effect was judged on the next day after the end of the test period. The evaluation criteria are as follows.

◎: 8 or more panelists recognized that the rough skin was improved.
○: 6 or more and less than 8 panelists recognized that the rough skin was improved.
△: 3 or more and less than 6 panelists recognized that the rough skin was improved.
×: less than 3 panelists recognized that the rough skin was improved.

"Evaluation (5): Skin Irritation Test"

A 24-hour occlusive patch test was performed on the medial side of the upper arm of 10 panelists, and the average value was calculated based on the following criteria.

0...No abnormality was observed.
1...Slight redness was observed.
2...Redness was observed.
3...Redness and papules were observed.

The evaluation criteria for the "skin irritation test" are as follows.

◎: Average value of 10 panelists: 0 or higher and lower than 0.1
○: Average value of 10 panelists: 0.1 or higher and lower than 0.15
△: Average value of 10 panelists: 0.15 or higher and lower than 0.2

×: Average value of 10 panelists: 0.2 or higher

Initially, the present inventors evaluated the conductance, based on the above-described criteria, for respective 10% aqueous solutions of various moisturizers.

( Table 1 )

| compound | 30 minutes | 60 minutes | 120minutes, |
|---|---|---|---|
| $C(-CH_2-[(EO)_2/(PO)_9]-CH_3)_4$ | ◎ | ◎ | ◎ |
| $C(-CH_2-[(EO)_9/(PO)_3]-CH_3)_4$ | ◎ | ◎ | ◎ |
| $C(-CH_2-[(EO)_6/(PO)_6]-CH_3)_4$ | ◎ | ◎ | ◎ |
| ion-exchanged water | △ | × | × |
| 1,3-butylene glycol | △ | △ | △ |
| glycerin | ◎ | ○ | ○ |

From the results in Table 1, it was clarified that the polyoxyalkylene glycol/polyethylene glycol copolymer alkyl ether derivatives were superior in moisture retention compared with common moisturizers such as 1,3-butylene glycol and glycerin.

Thus, the present inventors further investigated the polyoxyalkylene glycol/polyethylene glycol copolymer alkyl ether derivatives.

Each polyoxyalkylene glycol/polyethylene glycol copolymer alkyl ether derivative that was used in the test was prepared according to the below-described Synthesis Example 1 or Synthesis Example 2. In the present invention, EO represents an oxyethylene group, PO represents an oxypropylene group, and [(EO)/(PO)] represents random-type bonding.

Synthesis Example 1 Synthesis Example of Random Polymer

Pentaerythritol/polyoxypropylene glycol (2 mol)/polyethylene glycol (9 mol) tetramethyl ether

Into an autoclave, 34 g of pentaerythritol and 3.1 g of potassium hydroxide, as a catalyst, were loaded. The air in the autoclave was replaced with dry nitrogen, and the catalyst was completely dissolved with stirring at 140 ˚C. Then, a mixture of 396 g of ethylene oxide and 116 g of propylene oxide was dropwise added with a dropping apparatus, and the mixture was stirred for 2 hours. Subsequently, 75 g of potassium hydroxide was loaded, the inside of the system was replaced with dry nitrogen, 50 g of methyl chloride was pressured in at 80 to 130 ˚C, and the reaction was carried out for 5 hours. Then, the reaction mixture was taken out from the autoclave, neutralized with hydrochloric acid to pH 6 to 7, and treated at 100 ˚C for 1 hour under a reduced pressure of -0.095 MPa (50 mmHg) in order to remove contained water. In addition, filtration was conducted to remove the salt formed after the treatment, and the pentaerythritol derivative represented by the above-described chemical formula 4 was obtained.

The hydroxyl value of the compound that was obtained by purifying a sample taken before the methyl chloride reaction was 107, and the hydroxyl value of the obtained compound of chemical formula 4 was 0.4. The ratio of the number of terminal hydrogen atoms and the number of the terminal methyl groups was 0.004; thus, the hydrogen atoms are almost completely replaced with methyl groups.

Synthesis Example 2 Synthesis of Sorbitol Derivative

Sorbitol/polyoxypropylene glycol (12 mol)/polyoxyethylene glycol (54 mol) hexamethyl ether

Into an autoclave, 182 g of sorbitol and 18 g of potassium hydroxide, as a catalyst, were loaded. The air in the autoclave was replaced with dry nitrogen, and the catalyst was completely dissolved with stirring at 140 ˚C. Then, a mixture of 2376 g of ethylene oxide and 696 g of propylene oxide was dropwise added with a dropping apparatus, and the mixture was stirred for 4 hours. Subsequently, 600 g of potassium hydroxide was loaded, the inside of the system was replaced with dry nitrogen, 700 g of methyl chloride was pressured in at 80 to 130 ˚C, and the reaction was carried out for 5 hours. Then, the reaction mixture was taken out from the autoclave, neutralized with hydrochloric acid to pH 6 to 7, and treated at 100 ˚C for 1 hour under a reduced pressure of -0.088 MPa (gauge pressure) in order to remove contained water. In addition, filtration was conducted to remove the salt formed after the treatment, and the sorbitol derivative was obtained.

The hydroxyl value of the compound that was obtained by purifying a sample taken before the methyl chloride reaction was 109, and the hydroxyl value of the obtained sorbitol derivative was 0.7. The ratio of the number of terminal hydrogen

atoms and the number of the terminal methyl groups was 0.006; thus, the hydrogen atoms are almost completely replaced with methyl groups.

Various polyoxyalkylene glycol/polyethylene glycol copolymer alkyl ether derivatives were prepared according to the each synthesis example described above, and their skin external preparations were evaluated with the use of the below-described basic composition for testing.

| (described basic composition for testing) | |
|---|---|
| Ethanol | 2 wt% |
| Glycerine | 5 |
| 1-3-butylene glycol | 5 |
| Nicotinamide | 0.3 |
| Sodium pyrrolidonecarboxylate | 0.5 |
| Purified water | remainder |

[Determination of R]

Initially, the present inventors investigated the correlation between R and the suitability as a skin external preparation. The results are shown in Table 2. All compounds are pentaerythritol derivatives, and $[(EO)_9/(PO)_2]$ was used as the section of EO and PO. The blending quantity of each compound was 5 mass % with respect to the quantity of the above-described basic composition.

( Table 2 )

| Compund | R | Smoothness Evaluation(1) | Non-stickness (2) | Moisturizing (3) | Rough skin (4) | Irritation (5) |
|---|---|---|---|---|---|---|
| 1 | H | × | △ | × | | ○ |
| 2 | $CH_3$ | ◎ | ◎ | ◎ | ◎ | ◎ |
| 3 | $C_2H_5$ | ◎ | ◎ | ◎ | ◎ | ◎ |
| 4 | $C_3H_7$ | ◎ | ◎ | ◎ | ◎ | ◎ |
| 5 | $C_4H_9$ | ◎ | ◎ | ○ | ○ | ○ |
| 6 | $C_5H_{11}$ | △ | △ | △ | △ | △ |

As is clear from Table 2, when the number of carbons in R was 1 to 4 (compound 2 to 5), an excellent moisturizing effect and an excellent feeling in use could be achieved.
On the other hand, when R was hydrogen (compound 1), the sticky feeling was strong. When $\underline{R^1}$ was C5 (compound 5), both moisture retention and the feeling in us e were not desirable.
Thus, it is necessary that R is a hydrocarbon group having 1 to 4 carbon atoms in the base of the present invention.

In the actual production, all Rs are not necessarily substituted by hydrocarb on groups. Therefore, the acceptable existence rate of unsubstituted (H) compounds wa s investigated. The rate of unsubstituted compounds was expressed by the ratio of the number of hydrogen atoms (Y) with respect to the number of hydrocarbon groups (X), namely, Y/X.
In Table 3, 1:2 = 5:95 means that the compound 1 and compound 2 were mixed in a ratio of 5:95 to adjust the value to the specified Y/X.

( Table 3 )

| Compund | R | Y/X | Non-stickness (Evaluation 2) |
|---|---|---|---|
| 1 | H | 1.000 | △ |
| 2 | $CH_3$ | 0.004 | ◎ |
| 1:2=5:95 | | 0.053 | ◎ |
| 1:2=20:80 | | 0.202 | ◎ |

As is clear from Table 3, even when there were unreacted compounds for R, no great effect was caused if the amount was small (Y/X = 0.053). However, if Y/X was 0.202, a sticky feeling was clearly generated. As a result of further detailed investigation by the present inventors, it was clarified that Y/X was desirably 0.15 or less.

[Oxyalkylene Group and Oxyethylene Group]

Next, the present inventors investigated the presence of oxyalkylene groups and oxyethylene groups and the suitability as a skin external preparation (blended in an amount of 5 mass % with respect to the above-described basic composition).
The results are shown in Table 4. Compounds 7 to 11, and 14 are all pentaerythritol derivatives and R is $CH_3$. The oxyalkylene groups and oxyethylene groups are randomly added.
( Table 4 )

| Compund | EO | PO | BO | Evaluation(1) | (2) | (3) | (4) | (5) |
|---|---|---|---|---|---|---|---|---|
| 7 | 10 | 0 | 0 | ○ | ○ | × | × | ○ |
| 8 | 9 | 2 | 0 | ◎ | ◎ | ◎ | ◎ | ◎ |
| 2 | 5 | 5 | 0 | ◎ | ◎ | ◎ | ◎ | ◎ |
| 9 | 3 | 7 | 0 | ◎ | ◎ | ◎ | ◎ | ◎ |
| 10 | 0 | 10 | 0 | ◎ | ◎ | ◎ | ◎ | ◎ |
| 11 | 20 | 20 | 0 | difficult of adjust (low water solubility) | | | | |
| 12 | formula (IV) | | | ○ | △ | × | × | △ |
| 14 | 9 | 0 | 2 | ◎ | ◎ | ◎ | ◎ | ◎ |

The structure of chemical formula (IV) is shown below.

(formula IV)

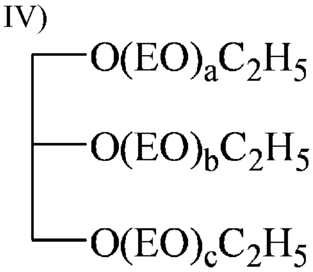

$(a+b+c)=9$ · · · (IV)

As is clear from Table 4, the presence of both oxyalkylene groups and oxyethylene groups is absolutely necessary for the moisture retaining property and the usability of the present invention. Because the effect of compound 12 represented by the above-described chemical formula (IV) is low, the effect is considered to be not a simple adjustment effect of hydrophilicity and hydrophobicity. Based on the further detailed investigation by the present inventors, it is clarified that the preferable percentage of the oxyethylene groups with respect to the sum of oxyalkylene groups and oxyethylene groups is about 20 mass % or higher, and more preferably 20 to 90 mass %.
In addition, the present inventors prepared a block polymer and a random polymer with the same number of oxyalkylene groups and the same number of oxyethylene groups and made a comparison.
( Table 5 )

| Compund | EO PO | Non-stickness (Evaluation 2) |
|---|---|---|
| 13 | $C[-CH_2O-(EO)_7(PO)_3-CH_3]_4$ | ○ |
| 2 | $C(-CH_2O-[(EO)_7/(PO)_3-CH_3)_4$ | ◎ |

As is clear from Table 5, an excellent feeling in use can be achieved especially in the case of the random polymer.

[Blending Quantity into Skin External Preparation]

Next, the present inventors further investigated the blending quantity of the polyoxyalkylene glycol/polyethylene glycol copolymer alkyl ether derivative of the present invention into the skin external preparation (above-described basic

composition).
]

( Table 6 )

| Compund 8 | 0 | 0.01 | 0.5 | 5.0 | 40.0 | 70.0 |
|---|---|---|---|---|---|---|
| Evaluation 1 | × | ○ | ◎ | ◎ | ◎ | ◎ |
| Evaluation 2 | × | ○ | ◎ | ◎ | ◎ | ◎ |
| Evaluation 3 | × | ○ | ◎ | ◎ | ◎ | ◎ |
| Evaluation 4 | × | ○ | ◎ | ◎ | ◎ | ◎ |
| Evaluation 5 | × | ○ | ◎ | ◎ | ◎ | ◎ |

From the results shown in Table 6, the addition effect of the compound of the present invention is observed from about 0.01 mass %, and it is especially prominent at 0.5 mass % or higher. However, slight stickiness starts to be generated at 70 mass %; thus the blending up to about 40 mass % is desirable.

[Stickiness Improving Effect]

The present inventors further investigated various blending modes of the polyoxyalkylene glycol/polyethylene glycol copolymer alkyl ether derivatives of the present invention. During the process, the present inventors discovered that the polyoxyalkylene glycol/polyethylene glycol copolymer alkyl ether derivatives had an improving effect of stickiness observed for moisturizers such as glycerin. As shown in Table 6, in the test segment where a polyoxyalkylene glycol/polyethylene glycol copolymer alkyl ether derivative was not blended, stickiness due to glycerin etc. was observed. On the other hand, when a polyoxyalkylene glycol/polyethylene glycol copolymer alkyl ether derivative was added, it improved the stickiness due to other moisturizers rather than simply suppressing the worsening of a sticky feeling.

[Promoting Effect of Percutaneous Absorption]

As shown in Table 7, the polyoxyalkylene glycol/polyethylene glycol copolymer alkyl ether derivative (I) and glycerin themselves have a moisturizing effect and rough skin improving effect. However, when used in combination with other moisturizers, the moisturizing effect and rough skin improving effect were synergistically and markedly improved.

( Table 7 )

| Composition | blending quantity (%) | | |
|---|---|---|---|
| polyoxyalkylene glycol/polyethylene glycol copolymer alkyl ether derivative* | 5 | - | 5 |
| Glycerin | - | 5 | - |
| Ion exchanged water | 95 | 95 | 90 |
| Conductance measurement evaluation | △ | △ | ◎ |
| * $C[-CH_2O-(EO)_9(PO)_2-CH_3]_4$ | | | |

Thus, the present inventors further investigated, in detail, the action of the polyoxyalkylene glycol/polyethylene glycol copolymer alkyl ether derivatives. As a result, it was found that the polyoxyalkylene glycol/polyethylene glycol copolymer alkyl ether derivatives of the present invention had a stratum corneum penetration promoting effect for other moisturizers such as glycerin. As shown in Fig. 1, the amount of penetrated glycerin increased about 40% when the polyoxyalkylene glycol/polyethylene glycol copolymer alkyl ether derivative of the present invention was used in combination with glycerin (sample 2) compared with the case of glycerin alone (control). The test method for Fig. 1 was as follows.
<u>1. Test Samples</u>

( Table 8 )

| Composition | Sample1(controlled) | sample2 |
|---|---|---|
| Glycerin | 10% | 10% |
| polyoxyalkylene glycol/polyethylene | | |

(continued)

| Composition | Sample1(controlled) | sample2 |
|---|---|---|
| glycol copolymer alkyl ether derivative* | 0 | 5 |
| Ion exchanged water | 90 | 85 |
| * $C[-CH_2O-(EO)_6(PO)_4-CH_3]_4$ | | |

2. Test Method

The test was conducted by the tape-stripping method. The tape-stripping method is as follows. After an agent is applied, the stratum corneum is stripped with a tape, and the agent concentration in the stratum corneum is determined. This method is generally used as a means for estimating the absorbed amount of the agent into the human skin. Specifically, the operation is conducted according to the below-described steps (1) to (7), and the penetration of glycerin was studied from the recovered amount of glycerin from the stratum corneum that was stripped with a tape. The test was conducted by 4 panelists, and the average value was used for evaluation.

(1) Medial side of both forearms of panelists is washed with soap.
(2) Sample application (20 mL/20 $cm^2$): On the medial side of the forearm, sample 1 is applied close to the wrist, and sample 2 is applied close to the elbow joint of the right arm. For the left arm, sample 1 and sample 2 are applied at the reversed positions of the right arm.
(3) Allowed to stand for 4 hours.
(4) Medial side of both forearms is washed with soap.
(5) The stratum corneum is stripped with a tape (8 layers).
(6) Glycerin is extracted from the tape with ion-exchanged water.
(7) Glycerin is quantitated (HPLC).

In addition, the present inventors conducted a similar investigation using xylitol as the moisturizer instead of glycerin. The test method was the same as the tape-stripping method for glycerin except for the (3) standing time of 6 hours. Used samples are shown in Table 9.

( Table 9 )

| Composition | Sample3 (controlled) | sample4 |
|---|---|---|
| Ethanol | 5% | 5% |
| Glycerin | 6 | 6 |
| Dipropylene Glycol | 5 | 5 |
| Xylitol | 3 | 3 |
| polyoxyalkylene glycol/polyethylene glycol copolymer alkyl ether derivative* | 0 | 5 |
| Ion exchanged water | 81 | 76 |
| * $C[-CH_2O-(EO)_9(PO)_2-CH_3]_4$ | | |

The results are shown in Fig. 2. As seen from Fig. 2, for all the four panelists A to D, the amount of penetrated xylitol into the stratum corneum was larger in the case of sample 4, in which a polyoxyalkylene glycol/polyethylene glycol copolymer alkyl ether derivative was blended, compared with sample 3 (control), in which a polyoxyalkylene glycol/ polyethylene glycol copolymer alkyl ether derivative was not blended.
From these results, it is suggested that the polyoxyalkylene glycol/polyethylene glycol copolymer alkyl ether derivative (I) promotes the percutaneous absorption of moisturizers such as glycerin and xylitol.

In order to further investigate the effect of the polyoxyalkylene glycol/polyethylene glycol copolymer alkyl ether derivative on hydrophilic agents other than moisturizers, the present inventors conducted a similar investigation using arbutin (hydroquinone-β-D-glucopyranoside). The test was done according to the tape-stripping method used for glycerin. The used samples and the results are shown in Table 10.

(Table 10)

| Composition | Sample5 (controlled) | sample4 |
|---|---|---|
| Glycerin | 10 | 10 |

(continued)

| Composition | Sample5 (controlled) | sample4 |
|---|---|---|
| polyoxyalkylene glycol/polyethylene glycol copolymer alkyl ether derivative* | 0 | 10 |
| Arbutin | 6 | 6 |
| Phenoxyethanol | 0.3 | 0.3 |
| Ethanol | 10 | 10 |
| Ion exchanged water | 73.7 | 63.7 |
| Arbutin recovered amount($\mu$g) | 0.9 | 4.6 |
| Glycerin recovered amount($\mu$g) | 129.7 | 156.8 |
| * $C[-CH_2O-(EO)_9(PO)_2-CH_3]_4$ | | |

As is clear from Table 10, the penetrated amounts of both arbutin and glycerin into the stratum corneum were larger in the case of sample 6, in which a polyoxyalkylene glycol/polyethylene glycol copolymer alkyl ether derivative was blended, compared with sample 5 (control), in which a polyoxyalkylene glycol/polyethylene glycol copolymer alkyl ether derivative was not blended.
From these results, it is suggested that the polyoxyalkylene glycol/polyethylene glycol copolymer alkyl ether derivative (I) also promotes the percutaneous absorption of whitening agents such as arbutin.
The action mechanism for the percutaneous absorption promoting effect of the polyoxyalkylene glycol/polyethylene glycol copolymer alkyl ether derivative of the present invention is not clear. However, it is speculated that the percutaneous absorption is promoted because the polyoxyalkylene glycol/polyethylene glycol copolymer alkyl ether derivative of the present invention lowers the affinity between the hydrophilic agents, such as moisturizers and whitening agents, and the base.
In theory, the larger the activity of an agent in the base, the larger the skin permeation rate of the agent. Therefore, in the region below the saturation solubility of the agent, it is possible to increase the partition into the skin by lowering the affinity between the agent and the base (by widening a difference in solubility parameter values between the agent and the base).
The polyoxyalkylene glycol/polyethylene glycol copolymer alkyl ether derivative (I) of the present invention is characterized in that it has high water solubility (solubility of 100% or more into water) as well as high oil solubility (solubility of 100% or more into ester oil). Such solubility characteristics are due to the chemical structure of the polyoxyalkylene glycol/polyethylene glycol copolymer alkyl ether derivative (I). The compound having both water solubility and oil solubility, having a function with which the solubility parameter of the base can be greatly changed when blended in a water-soluble base, and having excellent suitability as the base of skin external preparations hardly existed thus far.
Accordingly, it is inferred as follows: the solubility parameter of the base is greatly changed by the addition of a polyoxyalkylene glycol/polyethylene glycol copolymer alkyl ether derivative (I) to a water-soluble base, the affinity between the hydrophilic agent, such as moisturizer, and the base is significantly decreased; as a result, the stratum corneum penetration is enhanced. Thus, if an agent that is water-soluble, like moisturizer, and exhibits the effect through skin penetration is used as the hydrophilic agent, the effect enhancement can be expected. Such examples include water-soluble vitamins, amino acids, and whitening agents. The agents with high hydrophilicity are desirable as the agents for potential percutaneous absorption promotion. If the water/octanol partition coefficient (log P value), which exhibits the water solubility and oil solubility, is used as one indicator, the agents with 0 or lower are valid, and the agents with -1 or lower are more preferable though they are not limited to these. Examples of water-soluble agents with a log P value of -1.0 or lower include hydroquinone glycosides and derivatives thereof, ascorbic acid and derivatives thereof, and salicylic acid derivatives. The log P value that is defined, for example, in Chemical Reviews, vol. 71(6), 525 (1971) is a coefficient that represents polarity based on the material partition in water and octanol.
On the other hand, it is considered that the suppression of the percutaneous absorption of an oil-soluble agent having relatively high stratum corneum permeability is possible by blending a polyoxyalkylene glycol/polyethylene glycol copolymer alkyl ether derivative. For example, when an oil-soluble agent is solubilized or dispersed in a water-soluble base, a difference in the solubility parameters between the base and the agent becomes small by the addition of a polyoxyalkylene glycol/polyethylene glycol copolymer alkyl ether derivative, and the affinity between the agent and the base becomes high, resulting in the suppression of the stratum corneum penetration. Accordingly, the percutaneous absorption suppression is expected for oil-soluble agents (for example, preservatives and UV absorbers), for which no skin permeation is desirable, by blending a polyoxyalkylene glycol/polyethylene glycol copolymer alkyl ether derivative. As agents for potential percutaneous absorption suppression, the agents with a water/octanol partition coefficient (log P value) of 0.5 or higher are valid, and the agents with 1.0 or higher are more preferable though they are not limited to

these. Examples of oil-soluble agents with a log P value of 1.0 or higher include methylparaben, ethylparaben, butylparaben, phenoxyethanol, and octyl methoxycinnamate.

As described above, the percutaneous absorption promoting effect can be exhibited for hydrophilic agents such as moisturizers and whitening agents by blending a polyoxyalkylene glycol/polyethylene glycol copolymer alkyl ether derivative (I) of the present invention into a water-soluble base. On the other hand, the percutaneous absorption suppression is expected for oil-soluble agents such as preservatives and UV absorbers. In an oily base, it is inferred that the polyoxyalkylene glycol/polyethylene glycol copolymer alkyl ether derivatives can exhibit the opposite action. Thus, the polyoxyalkylene glycol/polyethylene glycol copolymer alkyl ether derivatives of the present invention can function as a percutaneous absorption control agent.

Because the properties of the polyoxyalkylene glycol/polyethylene glycol copolymer alkyl ether derivative (I) of the present invention can be adjusted by the numbers and percentages of EO chains and AO chains, there is also an advantage in that the suitability can be easily adjusted depending upon the target agent.

An advantage of the polyoxyalkylene glycol/polyethylene glycol copolymer alkyl ether derivatives (I) of the present invention will be described by comparing with the linear alkylene oxide derivatives. Generally, various liquid crystal phases of a surfactant with a high association number have high viscosity, and they are related with a frictional feeling and sticky feeling at the application time of cosmetics. The linear alkylene oxide derivatives have an action to change a high-order aggregate of surfactant to a microemulsion phase. This action is stronger in the case of the sterically-bulky polyoxyalkylene glycol/polyethylene glycol copolymer alkyl ether derivative (I) than the case of the linear alkylene oxide derivative. In order to compare this action, the phase diagram of the three-components system: $C(-CH_2-O-[(EO)_9/(PO)_2]-CH_3)_4$:polyoxyethylene (7 mol) oleyl ether = 3:7, liquid paraffin, and water; and the phase diagram of the three-components system: $CH_3O[(EO)_9/(PO)_2]CH_3$:polyoxyethylene (7 mol) oleyl ether = 3:7, liquid paraffin, and water were compared (refer to Fig. 3). As is clear from the figure, it is shown that $C(-CH_2-O-[(EO)_9/(PO)_2]-CH3)_4$ changes a hexagonal liquid crystal (H1) and cubic liquid crystal (I1), which appear in the phase diagram of $CH_3O[(EO)_9/(PO)_2]CH_3$, to a microemulsion phase (D or Wm) having low viscosity.

Formulation examples of the skin external preparation of the present invention will hereinafter be described.

Formulation example 1 : Cream

| | (% by weight) |
|---|---|
| A: Oil phase components | |
| Stearic acid | 10.0 |
| Stearyl alcohol | 4.0 |
| Butyl stearate | 8.0 |
| Monoglycerine ester stearate | 2.0 |
| Vitamine E acetate | 0.5 |
| Vitamine A palmitate | 0.1 |
| Macadamia oil | 1.0 |
| Tea seed oil | 3.0 |
| Perfume | 0.4 |
| Antiseptic | proper quantity |
| B: Water phase components | |
| Compound 2 | 5.0 |
| Glycerin | 4.0 |
| 1,2-Pentanediol | 3.0 |
| Sodium hyaluronate | 1.0 |
| Potasium hydroxide | 2.0 |
| Magnesium phosphate ascorbate | 0.1 |
| L-arginine hydrochloric acid | 0.01 |
| Trisodium edetate | 0.05 |
| Purified water | remainder |

(Preparation and Evaluation)

The oil phase (A) and the water phase (B) were completely dissolved by heating at 70 ˚C, respectively. (A) phase was added to (B) phase, and the mixture was emulsified with an emulsifier. The emulsified material was cooled with a heat exchanger to obtain a cream. The obtained cream was excellent in smoothness, there was no sticky feeling, and a moisturizing effect and rough skin improving effect were recognized.

Formulation example 2 : Cream

| | (% by weight) |
|---|---|
| A: Oil phase components | |
| Cetanol | 4.0 |
| Petrolatum | 7.0 |
| Isopropyl myristate | 8.0 |
| Squalane | 15.0 |
| Monoglycerine ester stearate | 2.2 |
| POE(20) sorbitan monostearate | 2.8 |
| Vitamine E nicotinate | 2.0 |
| Perfume | 0.3 |
| anti-oxidant aids | proper quantity |
| Antiseptic | proper quantity |
| B: Water phase components | |
| Compound 2 | 10.0 |
| Glycerin | 10.0 |
| Sodium hyaluronate | 0.02 |
| Dipropylene glycol | 4.0 |
| Sodium pyrrolidonecarboxylate | 1.0 |
| Disodium edetate | 0.01 |
| Purified water | remainder |

(Preparation and Evaluation)

Following Example 1, a cream was obtained. The obtained cream was excellent in smoothness, there was no sticky feeling, and a moisturizing effect and rough skin improving effect were recognized.

Formulation example 3 : Emulsion

| | (% by weight) |
|---|---|
| A: Oil phase components | |
| Squalane | 5.0 |
| Oleyl oleate | 3.0 |
| Petrolatum | 2.0 |
| Sorbitan sesquioleate ester | 0.8 |
| Polyoxyethylene oleyl ether (20EO) | 1.2 |
| Evening primrose oil | 0.5 |
| Perfume | 0.3 |
| Antiseptic | proper quantity |
| B: Water phase components | |
| Compound 9 | 8.0 |
| 1,3-butylene glycol | 4.5 |

(continued)

| | (% by weight) |
|---|---|
| Ethanol | 3.0 |
| Carboxyvinylpolymer | 0.2 |
| Potassium hydroxide | 0.1 |
| L-arginine L-glutamate | 0.01 |
| Edetic acid | 0.05 |
| Purified water | remainder |

(Preparation and Evaluation)

Following Example 1, a cream was obtained. The obtained cream was excellent in smoothness, there was no sticky feeling, and a moisturizing effect and rough skin improving effect were recognized.
Following Blending Example 1, a milky lotion was obtained. The obtained milky lotion was excellent in smoothness, there was no sticky feeling, and a moisturizing effect and rough skin improving effect were recognized.

Formulation example 4 : Foundation

| | (% by weight) |
|---|---|
| A: Oil phase components | |
| Cetanol | 3.5 |
| Deodorized lanolin | 4.0 |
| Jojoba oil | 5.0 |
| Petrolatum | 2.0 |
| Squalane | 6.0 |
| Monoglycerine ester stearate | 2.5 |
| POE(60) hydrogenated castor oil | 1.5 |
| POE(20) cetyl ether | 1.0 |
| Pyridoxine tripalmitate | 0.1 |
| Antiseptic | proper quantity |
| Perfume | 0.3 |
| B: Water phase components | |
| Compound 8 | 2.0 |
| propylene glycol | 10.0 |
| Blending powder | 12.0 |
| Trisodium hydroxyethyl ethylenediamine triacetate | 1.0 |
| Purified water | remainder |

(Preparation and Evaluation)

Following Blending Example 1, a foundation was obtained. The obtained foundation was excellent in smoothness, there was no sticky feeling, and a moisturizing effect and rough skin improving effect were recognized.

Formulation example 5 : Skin lotion

| | (% by weight) |
|---|---|
| A: Alcohol phase components | |
| Ethanol | 5.0 |
| POE oleyl alcohol ether | 2.0 |

(continued)

| | (% by weight) |
|---|---|
| Compound 11 | 3.0 |
| 2-ethylhexyl-P-dimethylamino benzoate | 0.18 |
| Perfume | 0.05 |
| B: Water phase components | |
| 1,3-butylene glycol | 9.5 |
| Sodium pyrrolidonecarboxylate | 0.5 |
| Nicotinamide | 0.3 |
| Glycerin | 5.0 |
| Dimorpholino pyridazinone | 0.5 |
| Purified water | remainder |

(Preparation and Evaluation)

The alcohol phase (A) was added to the water phase (B) and solubilized to obtain a lotion. The obtained lotion was excellent in smoothness, there was no sticky feeling, and a moisturizing effect and rough skin improving effect were recognized.

Embodiment 6 Water-in-oil type emulsified sunscreen

| | (% by weight) |
|---|---|
| (1) Ethylcellulose | 0.5 |
| (2) Ethyl alcohol | 4.0 |
| (3) Compound 2 | 2.0 |
| (4) 2-Ethylhexyl-P-methoxycinnamate | 5.0 |
| (5) Di (2-ethylhexyl) succinate alcohol | 22.0 |
| (6) Methyhydrogenpolysiloxane coated titanium dioxide | 6.0 |
| (7) Carboxymethyl cellulose | 1.0 |
| (8) Perfume | proper quantity |
| (9) Antiseptic | proper quantity |
| (10) Purified water | remainder |

(Preparation and Evaluation)

The components (2) - (3) were added to component (1), allowed to swell, and components (4) - (6) were added, heated with mixing, and sufficiently dispersed and dissolved. This dispersion liquid was kept at 70 ˚C and emulsified with a homomixer while gradually adding a mixed solution of component (7) and components (8) - (10), and a W/O emulsion-type sunscreen agent was obtained. The obtained W/O emulsion-type sunscreen agent was excellent in smoothness, there was no sticky feeling, and a moisturizing effect and rough skin improving effect were recognized.

Embodiment 7 Water-in-oil type emulsified sunscreen

| | (% by weight) |
|---|---|
| Component A | |
| Octylmethoxy cinnamate | 2.0 |
| Decamethylcyclopentasiloxane | 30.5 |
| Trimethylsiloxysilicate | 2.5 |
| Dimethly silicone | 5.0 |

(continued)

| | (% by weight) |
|---|---|
| POE polymethylsiloxane copolymer | 1.0 |
| Dimethylstearyl ammonium hectorite | 0.7 |
| Dextrin fatty acid coated titanium dioxide | |
| (average particle size 60nm) | 10.0 |
| Component B | |
| 1,3-butane diol | 5.0 |
| Compound 8 | 1.0 |
| Purified water | remainder |

(Preparation and Evaluation)

While component A was stirred with a homomixer, component B was gradually added and emulsified to obtain a W/O emulsion-type sunscreen agent. The obtained W/O emulsion-type sunscreen agent was excellent in smoothness, there was no sticky feeling, and a moisturizing effect and rough skin improving effect were recognized.

Embodiment 8 Water-in-oil type emulsified sunscreen

| | |
|---|---|
| (1) Behenic acid | 0.7 |
| (2) Isostearic acid | 0.7 |
| (3) Cetanol | 1.0 |
| (4) Liquid paraffin | 6.0 |
| (5) Dimethylpolysiloxane | 2.0 |
| (6) Glyceryl monostearate | 2.0 |
| (7) 2-Ethylhexyl-P-methoxycinnamate | 3.0 |
| (8) Polyethylene glycol 1500 | 5.0 |
| (9) Dipropylene glycol | 5.0 |
| (10) Titanium dioxide powder | 2.0 |
| (11) Potassium hydroxide | 0.1 |
| (12) Carboxyvinylpolymer | 1.0 |
| (13) Sodium hexametaphosphate | 0.05 |
| (14) Compound 11 | 2.0 |
| (15) Perfume | proper quantity |
| (16) Antiseptic | proper quantity |
| (17) Purified water | remainder |

(Preparation and Evaluation)

Components (1) - (7) and component (15) were mixed, respectively, heated at 80 ˚C and dissolved to obtain the oil phase. Components (8) - (14) and components (16) - (17) were heated at 70 to 75 ˚C and dispersed to obtain the water phase. The oil phase was gradually added to the water phase and emulsified with a homomixer.
The emulsified material was cooled to 30 ˚C with a heat exchanger, and an O/W emulsion-type sunscreen agent was obtained. The obtained O/W emulsion-type sunscreen agent was excellent in smoothness, there was no sticky feeling, and a moisturizing effect and rough skin improving effect were recognized.

Embodiment 9 Sunscreen Oil

| | |
|---|---|
| (1) 2-Ethylhexyl-P-methoxycinnamate | 3.0 |

(continued)

| | |
|---|---|
| (2) Compound 3 | 1.5 |
| (3) Hydrophobized titanium dioxide | 2.0 |
| (4) Hydrophobized zinc oxide | 1.0 |
| (5) Liquid paraffin | 51.0 |
| (6) Cetyloctanoate | 40.0 |
| (7) anti-oxidant aids | proper quantity |
| (8) Perfume | proper quantity |

(Preparation and Evaluation)

Each component of (1) - (8) was heated with stirring and cooled to obtain a sunscreen oil. The obtained sunscreen oil was excellent in smoothness, there was no sticky feeling, and a moisturizing effect and rough skin improving effect were recognized.

Embodiment 10 Water-in-oil type emulsified sunscreen

| | (% by weight) |
|---|---|
| (1) Ethylcellulose | 0.5 |
| (2) Ethyl alcohol | 4.0 |
| (3) Compound 2 | 2.0 |
| (4) 2-Ethylhexyl-P-methoxycinnamate | 5.0 |
| (5) Di (2-ethylhexyl) succinate alcohol | 22.0 |
| (6) Hydrophobized microparticulate titanium dioxide (average particle size 30nm) | 6.0 |
| (7) Carboxymethyl cellulose | 1.0 |
| (8) Perfume | proper quantity |
| (9) Antiseptic | proper quantity |
| (10) Purified water | remainder |

(Preparation and Evaluation)

Components (2) - (3) were added to component (1), allowed to swell sufficiently, and components (4) - (6) were added, heated with mixing, and sufficiently dispersed and dissolved. This sunscreen oil was kept at 70 ˚C and emulsified with a homomixer while a mixed solution of component (7) and components (8) - (10) were gradually added to obtain a W/O emulsion-type sunscreen agent. The obtained W/O emulsion-type sunscreen was excellent in smoothness, there was no sticky feeling, and a moisturizing effect and rough skin improving effect were recognized.

Formulation example 11 : Lotion mask

| | (% by weight) |
|---|---|
| A: Alcohol phase components | |
| Ethyl alcohol | 10.0 |
| PPG-13 decyltetradece-24 | 0.3 |
| Menthyl lactate | 0.004 |
| Antiseptic | proper quantity |
| Perfume | proper quantity |
| B: Water phase components | |
| Glycerin | 2.0 |
| Compound 9 | 3.0 |

(continued)

| | (% by weight) |
|---|---|
| Dipropylene glycol | 4.0 |
| Trehalose | 2.0 |
| Caustic potash | proper quantity |
| Purified water | remainder |

(Preparation and Evaluation)

The alcohol phase (A) was added to the water phase (B) to obtain a lotion. A lotion mask was obtained by further impregnating the lotion into a nonwoven cloth. The obtained lotion mask was excellent in smoothness, there was no sticky feeling, and a moisturizing effect and rough skin improving effect were recognized.

Formulation example 12 : Emulsion

| | (% by weight) |
|---|---|
| A: Oil phase components | |
| Squalane | 7.0 |
| Oleyl oleate | 2.0 |
| Petrolatum | 13.0 |
| Sorbitan sesquioleate ester | 0.8 |
| POE(20) oleyl ether | 1.2 |
| Perfume | proper quantity |
| Antiseptic | proper quantity |
| B: Water phase components | |
| Compound 14 | 3.0 |
| 1,3-butylene glycol | 1.0 |
| Ethanol | 4.0 |
| Carboxyvinylpolymer | 0.2 |
| Potassium hydroxide | 0.1 |
| Edetic acid | 0.05 |
| Purified water | remainder |

(Preparation and Evaluation)

Following Blending Example 1, a milky lotion was obtained. The obtained milky lotion was excellent in smoothness, there was no sticky feeling, and a moisturizing effect and rough skin improving effect were recognized.

## Claims

1. A skin external preparation comprising a polyoxyalkylene glycol/polyethylene glycol copolymer alkyl ether derivative represented by the below-described general formula (I):

(formula I) $Y\text{-}\{O(EO)_a / (AO)_{b\text{-}}R\}_k$ (I)

(In the formula, Y is the residue given by removing hydroxyl groups from a polyhydric alcohol having 3 to 6 hydroxyl groups, and k is the number of hydroxyl groups of the polyhydric alcohol. EO is an oxyethylene group; AO is an oxyalkylene group having 3 to 4 carbon atoms, and $1 \leqq a \leqq 70$, $1 \leqq b \leqq 70$. The percentage of the oxyethylene groups with respect to the sum of the oxyalkylene groups having 3 to 4 carbon atoms and the oxyethylene groups is 20 to 100 mass %. The oxyalkylene group having 3 to 4 carbon atoms and the oxyethylene group are added randomly. Rs are either identical to or different from each other, and they are hydrocarbon groups having 1 to 4 carbon atoms.)

**2.** A skin external preparation according to claim 1, in the above-described general formula (I), wherein Y is the residue of pentaerythritol and that the percentage of the oxyethylene groups with respect to the sum of the oxyalkylene groups having 3 to 4 carbon atoms and the oxyethylene groups is 50 to 80 mass %.

**3.** A skin external preparation according to claim 1 or 2, comprising 0.01 to 70 mass % of a polyoxyalkylene glycol/polyethylene glycol copolymer alkyl ether derivative represented by the above-described general formula (I).

**4.** A moisturizer comprising a polyoxyalkylene glycol/polyethylene glycol copolymer alkyl ether derivative represented by the above -described general formula (I).

**5.** A rough skin improving agent comprising a polyoxyalkylene glycol/polyethylene glycol copolymer alkyl ether derivative represented by the above -described general formula (I).

**6.** A stickiness improving agent comprising a polyoxyalkylene glycol/polyethylene glycol copolymer alkyl ether derivative represented by the above -described general formula (I).

**7.** A percutaneous absorption promoter comprising a polyoxyalkylene glycol/polyethylene glycol copolymer alkyl ether derivative represented by the above -described general formula (I).

**8.** A skin external preparation comprising a polyoxyalkylene glycol/polyethylene glycol copolymer alkyl ether derivative represented by the general formula (I), and a hydrophilic agent, and said polyoxyalkylene glycol/polyethylene glycol copolymer alkyl ether derivative is a percutaneous absorption promoter of the hydrophilic agent.

**9.** A skin external preparation according to claim 8, wherein the hydrophilic agent is a moisturizer.

**10.** A skin external preparation according to claim 9, wherein the moisturizer is glycerin or xylitol.

**11.** A skin external preparation according to claim 8, wherein the hydrophilic agent is at least one selected from the group consisting of hydroquinone derivatives and ascorbic acid derivatives.

**12.** A percutaneous absorption control agent comprising a polyoxyalkylene glycol/polyethylene glycol copolymer alkyl ether derivative represented by the above -described general formula (I).

FIG.1

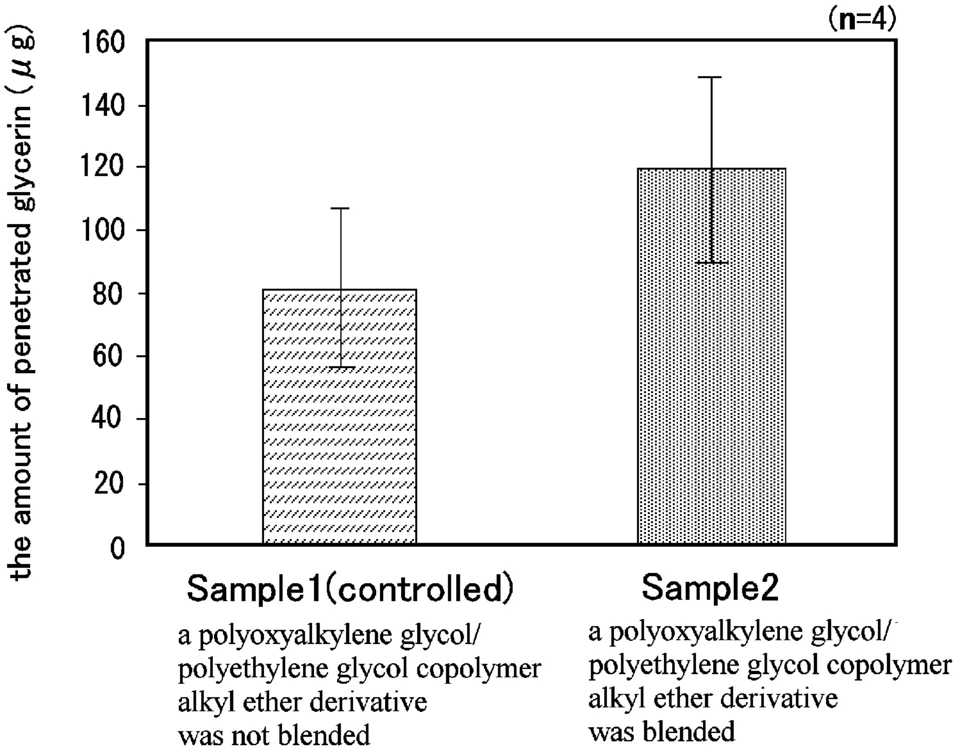
(n=4)
the amount of penetrated glycerin (μg)
160
140
120
100
80
60
40
20
0
Sample1(controlled)
a polyoxyalkylene glycol/
polyethylene glycol copolymer
alkyl ether derivative
was not blended
Sample2
a polyoxyalkylene glycol/
polyethylene glycol copolymer
alkyl ether derivative
was blended

FIG.2

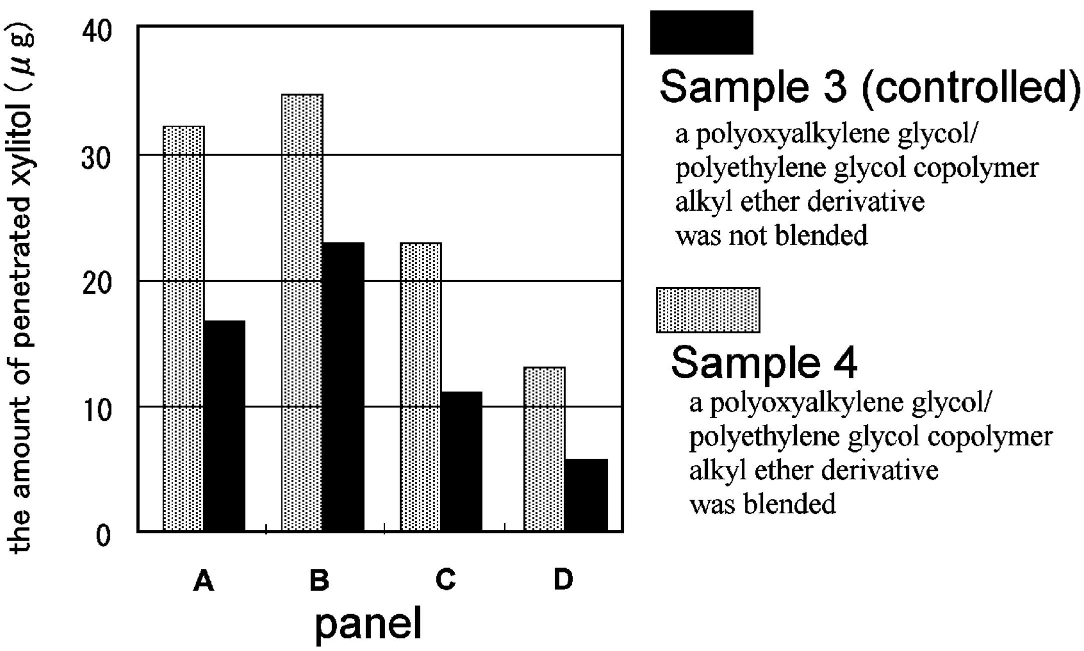
the amount of penetrated xylitol (μg)
40
30
20
10
0
A
B
C
D
panel
Sample 3 (controlled)
a polyoxyalkylene glycol/
polyethylene glycol copolymer
alkyl ether derivative
was not blended
Sample 4
a polyoxyalkylene glycol/
polyethylene glycol copolymer
alkyl ether derivative
was blended

FIG.3

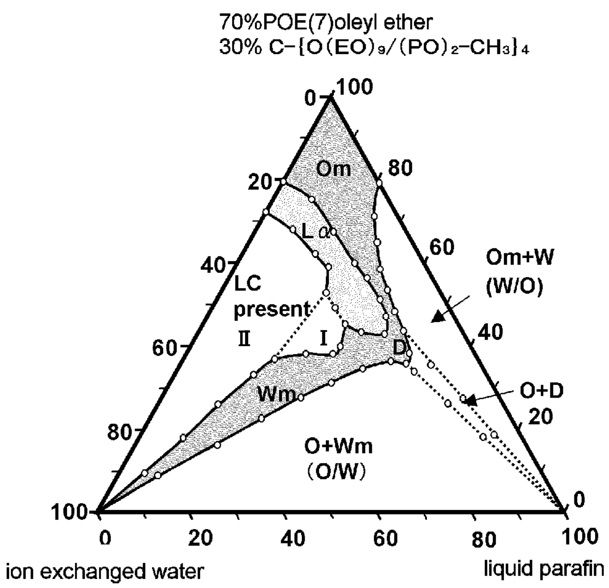
70%POE(7)oleyl ether
30% C-{O(EO)9/(PO)2-CH3}4
0
100
Om
80
20
Lα
60
Om+W
(W/O)
40
LC
present
II
I
D
40
60
O+D
Wm
20
80
O+Wm
(O/W)
0
100
0
20
40
60
80
100
ion exchanged water
liquid parafin

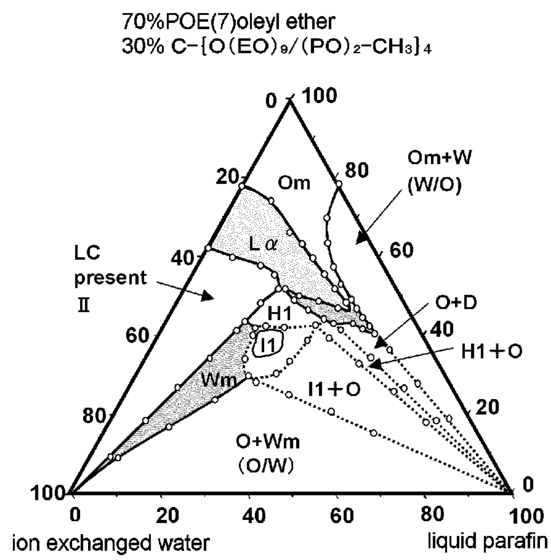
70%POE(7)oleyl ether
30% C-{O(EO)9/(PO)2-CH3}4
0
100
80
Om+W
(W/O)
20
Om
Lα
LC
present
II
40
60
H1
O+D
I1
40
60
H1+O
Wm
I1+O
20
80
O+Wm
(O/W)
100
0
0
20
40
60
80
100
ion exchanged water
liquid parafin

**INTERNATIONAL SEARCH REPORT**

International application No.
PCT/JP2007/060454

A. CLASSIFICATION OF SUBJECT MATTER
*A61K8/86*(2006.01)i, *A61K47/34*(2006.01)i, *A61Q1/02*(2006.01)i, *A61Q17/04*(2006.01)i, *A61Q19/00*(2006.01)i

According to International Patent Classification (IPC) or to both national classification and IPC

B. FIELDS SEARCHED

Minimum documentation searched (classification system followed by classification symbols)
A61K8/86, A61K47/34, A61Q1/02, A61Q17/04, A61Q19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2005-162782 A (Sanyo Chemical Industries, Ltd.),<br>23 June, 2005 (23.06.05),<br>Abstract; Claims 1, 5; Par. Nos. [0014], [0019], [0040] to [0041]<br>(Family: none) | 1-3,6,8-11<br>4-5,7,12 |
| Y<br>A | JP 2004-83541 A (Shiseido Co., Ltd.),<br>18 March, 2004 (18.03.04),<br>Abstract; tables 2 to 4<br>& CN 1408339 A & EP 1283031 A2<br>& JP 3660656 B2 & JP 3667707 B2<br>& KR 20030027644 A & US 2003/180335 A1 | 1-6,8-11<br>7,12 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
06 August, 2007 (06.08.07)

Date of mailing of the international search report
21 August, 2007 (21.08.07)

Name and mailing address of the ISA/
Japanese Patent Office

Facsimile No.

Authorized officer

Telephone No.

Form PCT/ISA/210 (second sheet) (April 2005)

**INTERNATIONAL SEARCH REPORT**

International application No.
PCT/JP2007/060454

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2001-322925 A (Shiseido Co., Ltd.),<br>20 November, 2001 (20.11.01),<br>Abstract; Claim 3 (particularly, chemical formula (2)); Claim 6; Par. No. [0005]; table 6; example 7<br>(Family: none) | 1-6,8-10<br>7,11-12 |
| Y<br>A | JP 2002-121129 A (Shiseido Co., Ltd.),<br>23 April, 2001 (23.04.01),<br>Abstract; Claim 3 (particularly, chemical formula (2)); Claim 6; Par. No. [0003]; table 5; examples 5, 10<br>(Family: none) | 1-3,6,8-11<br>4-5,7,12 |
| P,X | JP 2006-306857 A (NOF Corp.),<br>09 November, 2006 (09.11.06),<br>Abstract; Claims; synthesis examples 1 to 9; examples 1 to 15<br>(Family: none) | 1-6,8-10 |
| A | WO 2001/91709 A (PROCTER & GAMBLE),<br>06 December, 2001 (06.12.01),<br>Abstract<br>& AT 346654 A & AU 1460201 A<br>& AU 6501001 A & CN 1217645 C<br>& DE 60124904 A & EP 1284712 A2<br>& JP 2004-501112 A & MX PA02011886 A<br>& US 6709648 B2 & WO/0191708 A1 | 1-12 |
| A | JP 11-100312 A (Shiseido Co., Ltd.),<br>13 April, 1999 (13.04.99),<br>Abstract<br>(Family: none) | 1-12 |
| A | JP 2001-2591 A (Dai-Ichi Kogyo Seiyaku Co., Ltd.),<br>09 January, 2001 (09.01.01),<br>Abstract<br>(Family: none) | 7,12 |

Form PCT/ISA/210 (continuation of second sheet) (April 2005)

**INTERNATIONAL SEARCH REPORT**

International application No.
PCT/JP2007/060454

The expression "Japanese Patent No. 3660650" (Patent Document 2) in paragraph No. [0005] in the description is a clerical error and is correctly to read --Japanese Patent No. 3660656--.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2006142384 A
- JP 2006269122 A
- JP 3658012 B
- JP 3660650 B
- JP 3667707 B

### Non-patent literature cited in the description

- *Chemical Reviews,* 1971, vol. 71 (6), 525